# EUROPÄISCHE PATENTSCHRIFT

(11) **EP 4 294 996 B1**
(45) Veröffentlichungstag und Bekanntmachung des Hinweises auf die Patenterteilung: **14.01.2026**
(21) Anmeldenummer: 22708860.6
(22) Anmeldetag: 15.02.2022
(51) Int. Cl.: E04G 21/02, E04G 21/04, B28C 5/42, B28C 7/02, B28C 7/16, G01K 11/00, G01N 22/04

(54) **BAUSTOFFVORRICHTUNG UND VERWENDUNG MINDESTENS EINES TEILSENSORS**
BUILDING MATERIAL APPARATUS AND USE OF AT LEAST ONE PART SENSOR
APPAREIL POUR MATÉRIAU DE CONSTRUCTION ET UTILISATION D'AU MOINS UN CAPTEUR DE PARTIE

(30) Priorität: 16.02.2021 DE 102021201456
(43) Veröffentlichungstag der Anmeldung: 27.12.2023
(73) Patentinhaber: Putzmeister Engineering GmbH, 72631 Aichtal (DE)
(72) Erfinder: SCHMIDT, Harald, 87700 Memmingen (DE)
(74) Vertreter: Patentanwälte Ruff, Wilhelm, Beier, Dauster & Partner mbB
(86) Internationale Anmeldenummer: PCT/EP2022/053597
(87) Internationale Veröffentlichungsnummer: WO 2022/175232

(56) Entgegenhaltungen:
- EP-A1- 1 447 192
- WO-A2-2011/004982
- CN-U- 204 645 658
- CN-U- 209 670 357
- DE-A1- 19 908 151
- DE-U1- 202010 012 841

## Beschreibung

### ANWENDUNGSGEBIET UND STAND DER TECHNIK

Die Erfindung bezieht sich auf eine Baustoffvorrichtung aufweisend mindestens einen Teilsensor und eine Verwendung mindestens eines solchen Teilsensors, insbesondere einer solchen Baustoffvorrichtung.

Die CN 204 645 658 U offenbart ein automatisches Zementbewässerungssystem, das auf einer Fernbedienung basiert.

Die EP 1 447 192 A1 offenbart ein Verfahren und eine Vorrichtung zum Gießen von Beton.

Die WO 2011/004982 A2 offenbart ein sicheres Betriebssystem und ein sicheres Betriebsverfahren für Geräte zur Betonversorgung.

Die CN 209 670 357 U offenbart ein einfaches Betontransportgerät für eine Baustelle.

Die DE 199 08 151 A1 offenbart ein Verfahren zum Herstellen von aus Beton bestehenden Elementen.

### AUFGABE UND LÖSUNG

Der Erfindung liegt als Aufgabe die Bereitstellung einer Baustoffvorrichtung aufweisend mindestens einen Teilsensor und einer Verwendung mindestens eines solchen Teilsensors, insbesondere einer solchen Baustoffvorrichtung, zugrunde, die jeweils verbesserte Eigenschaften aufweisen.

Die Erfindung löst die Aufgabe durch die Bereitstellung einer Baustoffvorrichtung mit den Merkmalen des Anspruchs 1 und einer Verwendung mit den Merkmalen des Anspruchs 10. Vorteilhafte Weiterbildungen und/oder Ausgestaltungen der Erfindung sind in den abhängigen Ansprüchen beschrieben.

Die erfindungsgemäße Baustoffvorrichtung umfasst bzw. weist mindestens, insbesondere nur, ein, insbesondere einziges, Baustofftransportteil, zur Nachfolge bzw. nachfolgend bzw. nach und/oder verschieden von einer Baustofftrommel, und mindestens, insbesondere nur, einen, insbesondere einzigen und/oder elektrischen, Teilsensor auf. Das Baustofftransportteil ist zum Transport von, insbesondere noch, mindestens, insbesondere nur, teilweise, insbesondere vollständig, auszuhärtendem bzw. nicht ausgehärtetem bzw. nicht erhärtetem Baustoff, insbesondere in dem Baustofftransportteil und/oder nachfolgend bzw. nach der Baustofftrommel, ausgebildet bzw. konfiguriert. Mindestens der Teilsensor ist, insbesondere vollständig und/oder unmittelbar bzw. direkt, an oder in dem Baustofftransportteil zur, insbesondere automatischen, Erfassung, insbesondere nur, mindestens einer intensiven Größe, insbesondere eines Werts der intensiven Größe, oder mindestens einer mindestens der intensiven Größe korrespondierenden bzw. entsprechenden Größe, insbesondere eines Werts der korrespondierenden Größe, des Baustoffs, insbesondere transportiert, in dem Baustofftransportteil angeordnet bzw. angebracht, insbesondere befestigt, und ausgebildet bzw. konfiguriert.

Dies, insbesondere die Anordnung des Teilsensors an oder in dem Baustofftransportteil nachfolgend der Baustofftrommel, ermöglicht eine gute Zugänglichkeit zu dem Teilsensor und/oder einen geringen Verschleiß des Teilsensors und/oder einen geringen Verbrauch von Bauraum durch den Teilsensor, insbesondere im Unterschied zu einer nicht-erfindungsgemäßen Anordnung eines Teilsensors an und/oder in einer Baustofftrommel. Somit ermöglicht dies eine einfache Reinigung, eine einfache Wartung, eine einfache Reparatur und/oder einen einfachen Austausch des Teilsensors. Zusätzlich oder alternativ ermöglicht, insbesondere somit, dies eine zuverlässige Erfassung der intensiven Größe oder der korrespondierenden Größe, insbesondere mittels des Teilsensors. Zusätzlich oder alternativ kann die intensive Größe oder die korrespondierende Größe interessant und/oder hilfreich, insbesondere für eine Rückmeldung, für eine nächste Dosierung bzw. Mischung bzw. Charge von Baustoff sein.

Insbesondere kann sich die intensive Größe oder die korrespondierende Größe in der Baustofftrommel verändern, z.B. aufgrund einer kalten Außentemperatur im Winter oder einer warmen Außentemperatur im Sommer, insbesondere von einer Baustoffmischanlage zu einer Baustelle, insbesondere bei einem Transport.

Die Baustofftrommel kann für ein bzw. eines Baustofftransportfahrzeugs sein.

Das Baustofftransportteil kann vorhergehend einer Schalung sein.

Das Baustofftransportteil kann zum Transport von dem Baustoff in, insbesondere unmittelbarem/r bzw. direktem/r, Kontakt bzw. Berührung mit dem Baustoff sein.

Der mindestens teilweise auszuhärtende Baustoff kann ein Dickstoff und/oder ein Gemisch aus flüssigen und festen Bestandteilen sein.

Der Teilsensor kann außerhalb der Baustofftrommel bzw. braucht oder kann nicht an und/oder in der Baustofftrommel angeordnet sein.

Der Teilsensor braucht oder kann nicht zur Erfassung einer extensiven Größe oder einer der extensiven Größe korrespondierenden Größe des Baustoffs ausgebildet sein.

Für den Begriff intensive Größe kann der Begriff stoffliche Eigenschaft bzw. Stoffeigenschaft synonym verwendet werden.

Die Baustoffvorrichtung ist eine Betonvorrichtung. Zusätzlich ist das Baustofftransportteil ein Betontransportteil zur Nachfolge bzw. nachfolgend bzw. nach und/oder verschieden von einer Betontrommel eines Betontransportfahrzeugs. Weiter zusätzlich ist das Baustofftransportteil zum Transport von, insbesondere noch und/oder vollständig, auszuhärtendem Beton bzw. Frischbeton bzw. Transportbeton, insbesondere in dem Betontransportteil und/oder nachfolgend bzw. nach der Betontrommel, ausgebildet bzw. konfiguriert. Weiter zusätzlich ist mindestens der Teilsensor zur Erfassung der intensiven Größe oder der korrespondierenden Größe des Betons ausgebildet. In anderen Worten: Der Baustoff kann Beton sein. Für einen derartigen Baustoff kann die zuverlässige Erfassung der intensiven Größe oder der korrespondierenden Größe besonders vorteilhaft sein und/oder eine Rückmeldung, insbesondere für eine nächste Dosierung bzw. Mischung bzw. Charge, kann besonders interessant und/oder hilfreich sein.

Das Baustofftransportteil ist ein Auslauftrichter des Baustofftransportfahrzeugs, insbesondere zur Nachfolge bzw. nachfolgend bzw. nach der Baustofftrommel, eine Schurre des Baustofftransportfahrzeugs, insbesondere zur Nachfolge bzw. nachfolgend bzw. nach dem Auslauftrichter, ein Baustoffkübel in Form eines Krankübels, insbesondere zur Nachfolge bzw. nachfolgend bzw. nach dem Auslauftrichter und/oder der Schurre, ein Einlauftrichter einer Baustoffpumpe, insbesondere zur Nachfolge bzw. nachfolgend bzw. nach dem Auslauftrichter und/oder der Schurre, oder ein Endschlauch der Baustoffpumpe oder einer Baustoffverteilervorrichtung in Form eines Baustoffverteilermasts, insbesondere zur Nachfolge bzw. nachfolgend bzw. nach der Baustoffpumpe. Dies, insbesondere die Anordnung des Teilsensors an oder in einem derartigen Baustofftransportteil, ermöglicht eine besonders gute Zugänglichkeit zu dem Teilsensor. Insbesondere kann die Schurre eine Auslaufschurre sein. Zusätzlich oder alternativ kann der Baustoffkübel ein Betonkübel sein. Weiter zusätzlich oder alternativ kann die Baustoffpumpe eine Dickstoff- und/oder Betonpumpe und/oder eine mobile Baustoffpumpe, insbesondere eine Autobaustoffpumpe, insbesondere eine Autobetonpumpe, sein. Weiter zusätzlich oder alternativ kann der Baustoffverteilermast ein Faltmast sein.

Die Baustoffvorrichtung umfasst bzw. weist eine, insbesondere elektrische, Bestimmungseinrichtung auf. Die Bestimmungseinrichtung ist zur, insbesondere automatischen, Bestimmung einer Dosier- bzw. Misch- bzw. Rezeptgröße, insbesondere eines Werts der Dosiergröße, basierend auf mindestens der erfassten intensiven Größe oder der erfassten korrespondierenden Größe und einer Information über einen Anordnungsort des Teilsensors und/oder eine Art bzw. einen Typ des Baustofftransportteils und einer Anordnungsort- und/oder Baustofftransportteil-spezifischen Kennformel und/oder eines Anordnungsort- und/oder Baustofftransportteil-spezifischen Kennfelds ausgebildet bzw. konfiguriert. Die Information ermöglicht die Bestimmung der Dosiergröße mittels der Anordnungsort- und/oder Baustofftransportteil-spezifischen Kennformel und/oder eines Anordnungsort- und/oder Baustofftransportteil-spezifischen Kennfelds. Zusätzlich oder alternativ ermöglicht die Dosiergröße eine, insbesondere aussagekräftige, Rückmeldung für eine nächste Dosierung bzw. Mischung bzw. Charge von Baustoff. Weiter zusätzlich oder alternativ kann die Bestimmungseinrichtung einen Computer aufweisen, insbesondere sein.

Mindestens der Teilsensor ist zur Erfassung, insbesondere nur, einer Feuchtigkeit, insbesondere eines Werts der Feuchtigkeit, bzw. eines Wassergehalts und/oder einer Temperatur, insbesondere eines Werts der Temperatur, des Baustoffs ausgebildet bzw. konfiguriert. In anderen Worten: Die intensive Größe kann die Feuchtigkeit und/oder die Temperatur sein. Eine zuverlässige Erfassung einer derartigen intensiven Größe kann besonders vorteilhaft sein, insbesondere für ein gutes Aushärten bzw. Binden des Baustoffs. Zusätzlich oder alternativ kann eine derartige intensive Größe besonders interessant und/oder hilfreich, insbesondere für eine Rückmeldung, für eine nächste Dosierung bzw. Mischung bzw. Charge von Baustoff sein, insbesondere für ein gutes Aushärten bzw. Binden des Baustoffs.

Die Baustoffvorrichtung umfasst bzw. weist das Baustofftransportfahrzeug in Form eines Fahrmischers, die Baustoffpumpe oder, die Baustoffverteilervorrichtung in Form des Baustoffverteilermasts auf. Insbesondere ist die Baustoffvorrichtung das Baustofftransportfahrzeug, insbesondere der Fahrmischer, die Baustoffpumpe und/oder die Baustoffverteilervorrichtung, insbesondere der Baustoffverteilermast. Zusätzlich kann die Baustoffvorrichtung die Baustofftrommel in Form einer Mischtrommel aufweisen bzw. umfassen. Dies ermöglicht, insbesondere ermöglichen/ermöglicht das Baustofftransportfahrzeug und/oder die Baustofftrommel, einen Transport von dem Baustoff zu einer Baustelle, insbesondere von einer Betonmischanlage. Zusätzlich oder alternativ ermöglicht dies, insbesondere ermöglichen/ermöglicht die Baustoffpumpe und/oder die Baustoffverteilervorrichtung, einen Transport von dem Baustoff auf der Baustelle, insbesondere von dem Baustofftransportfahrzeug und/oder zu einer Schalung. Insbesondere kann der Fahrmischer ein fahrbarer Betonmischer bzw. ein Betonmischfahrzeug und/oder ein Trommelmischer, insbesondere ein Umkehrtrommelmischer, sein. Zusätzlich oder alternativ kann die Baustoffpumpe eine Dickstoff- und/oder Betonpumpe und/oder eine mobile Baustoffpumpe, insbesondere eine Autobaustoffpumpe, insbesondere eine Autobetonpumpe, sein. Weiter zusätzlich oder alternativ kann der Baustoffverteilermast ein Faltmast sein. Weiter zusätzlich oder alternativ kann die Mischtrommel eine Fahrmischertrommel sein.

In einer Weiterbildung der Erfindung ist mindestens der Teilsensor zur Erfassung der intensiven Größe oder der korrespondierenden Größe mittels Mikrowellen, insbesondere MikrowellenStrahlung, bzw. basierend auf einem Mikrowellen-Erfassungs- bzw. Messprinzip ausgebildet bzw. konfiguriert. Dies ermöglicht die Erfassung der Feuchtigkeit und/oder der Temperatur. Im Übrigen wird auf die Fachliteratur verwiesen.

In einer Weiterbildung der Erfindung umfasst bzw. weist die Baustoffvorrichtung eine Flachsonde, insbesondere eine Tellersonde oder eine Rechtecksonde, auf. Die Flachsonde umfasst bzw. weist den Teilsensor auf. Dies ermöglicht eine Kostengünstigkeit der Baustoffvorrichtung, insbesondere im Unterschied zu einer, insbesondere nicht-erfindungsgemäßen, Stabsonde. Zusätzlich oder alternativ ermöglicht dies die Anordnung des Teilsensors an und/oder in dem Baustofftransportteil. Insbesondere kann die Flachsonde, insbesondere vollständig und/oder unmittelbar bzw. direkt, an und/oder in dem Baustofftransportteil angeordnet bzw. angebracht, insbesondere befestigt, sein.

In einer Weiterbildung der Erfindung umfasst bzw. weist das Baustofftransportteil ein Prallblech auf. Der Teilsensor ist, insbesondere vollständig und/oder unmittelbar bzw. direkt, auf und/oder in dem Prallblech angeordnet bzw. angebracht, insbesondere befestigt. Dies ermöglicht eine besonders gute Erfassung der intensiven Größe oder der korrespondierenden Größe, insbesondere mittels des Teilsensors.

In einer Weiterbildung der Erfindung umfasst bzw. weist die Baustoffvorrichtung eine, insbesondere elektrische, Ermittlungseinrichtung, insbesondere eine Erfassungseinrichtung, auf. Die Ermittlungseinrichtung ist zur, insbesondere automatischen, Ermittlung, insbesondere die Erfassungseinrichtung zur Erfassung, insbesondere nur, einer Konsistenz, insbesondere eines Werts der Konsistenz, und/oder einer Viskosität, insbesondere eines Werts der Viskosität, oder einer der Konsistenz und/oder der Viskosität korrespondierenden bzw. entsprechenden Größe, insbesondere eines Werts der korrespondierenden Größe, des Baustoffs, insbesondere in der Baustofftrommel, insbesondere einer Antriebsgröße, insbesondere eines Werts der Antriebsgröße, einer Antriebseinrichtung zum Drehantrieb bzw. zum Antrieb zur Drehung der Baustofftrommel, ausgebildet bzw. konfiguriert. Eine derartige Größe kann zu der intensiven Größe oder der korrespondierenden Größe, insbesondere der Feuchtigkeit und/oder der Temperatur, komplementär und/oder, insbesondere somit, besonders interessant und/oder hilfreich, insbesondere für eine Rückmeldung, für eine nächste Dosierung bzw. Mischung von Baustoff sein. Insbesondere können/kann die Konsistenz und/oder die Viskosität oder die korrespondierende Größe von der intensiven Größe oder der korrespondierenden Größe, insbesondere mittels mindestens des Teilsensors erfasst, verschieden und/oder zu dieser komplementär sein. Zusätzlich oder alternativ kann die Ermittlungseinrichtung von mindestens dem Teilsensor verschieden sein. Weiter zusätzlich oder alternativ kann die Ermittlungseinrichtung zur, insbesondere automatischen, Ermittlung der Konsistenz und/oder der Viskosität basierend auf der Antriebsgröße ausgebildet bzw. konfiguriert sein. Weiter zusätzlich oder alternativ kann die Ermittlungseinrichtung einen Computer aufweisen, insbesondere sein. Weiter zusätzlich oder alternativ kann die Antriebsgröße einen Hydraulikantriebsdruck, insbesondere der Antriebseinrichtung in Form einer Hydraulikantriebseinrichtung, ein Antriebsdrehmoment und/oder einen Antriebsstrom, insbesondere einer elektrischen Antriebseinrichtung, aufweisen, insbesondere sein. Weiter zusätzlich oder alternativ kann die Baustoffvorrichtung die Antriebseinrichtung aufweisen bzw. umfassen. Im Übrigen wird auf die Fachliteratur verwiesen.

In einer Ausgestaltung der Erfindung ist die Bestimmungseinrichtung zur, insbesondere automatischen, Bestimmung der Dosier- bzw. Misch- bzw. Rezeptgröße, insbesondere eines Werts der Dosiergröße, basierend auf der ermittelten Konsistenz und/oder der ermittelten Viskosität oder der ermittelten korrespondierenden Größe ausgebildet bzw. konfiguriert.

In einer Weiterbildung, insbesondere einer Ausgestaltung, der Erfindung umfasst bzw. weist die Baustoffvorrichtung eine, insbesondere elektrische, Datenübertragungseinrichtung auf. Die Datenübertragungseinrichtung ist zur, insbesondere automatischen und/oder kabellosen, Datenübertragung mindestens der erfassten intensiven Größe oder der erfassten korrespondierenden Größe, insbesondere und der ermittelten Konsistenz und/oder der ermittelten Viskosität oder der ermittelten korrespondierenden Größe und/oder der Information über den Anordnungsort des Teilsensors oder die Art bzw. den Typ des Baustofftransportteils oder einer, insbesondere der, auf der intensiven Größe, insbesondere und der Konsistenz und/oder der Viskosität, oder der korrespondierenden Größe, insbesondere und der Information über den Anordnungsort des Teilsensors oder die Art des Baustofftransportteils, basierenden Dosier- bzw. Misch- bzw. Rezeptgröße, insbesondere zu einem Datenmanagementsystem, insbesondere für eine Baustoffmischanlage bzw. ein Baustoffmischwerk, ausgebildet bzw. konfiguriert. Die Information ermöglicht die Bestimmung einer, insbesondere der, Dosiergröße, insbesondere mittels einer Anordnungsort- und/oder Baustofftransportteil-spezifischen Kennformel und/oder eines Anordnungsort- und/oder Baustofftransportteil-spezifischen Kennfelds. Zusätzlich oder alternativ ermöglicht die Datenübertragung eine, insbesondere aussagekräftige, Rückmeldung für eine nächste Dosierung bzw. Mischung von Baustoff. Insbesondere kann die Baustoffmischanlage stationär und/oder eine Betonmischanlage bzw. ein Betonmischwerk sein.

In einer Weiterbildung der Erfindung umfasst bzw. weist die Baustoffvorrichtung mindestens einen, insbesondere elektrischen, Nebenverbraucher, insbesondere eine Kontroll- bzw. Steuer- bzw. Regeleinrichtung zur, insbesondere automatischen, Kontrolle bzw. Steuerung bzw. Regelung der Baustofftrommel, auf. Der Nebenverbraucher und der Teilsensor sind, insbesondere miteinander, zur, insbesondere automatischen und/oder elektrischen, Energie- und/oder Datenübertragung, insbesondere durch eine, insbesondere die und/oder elektrische und/oder gemeinsame, Energieversorgungs- und/oder Datenübertragungseinrichtung, insbesondere nur, berührungsbehaftet, insbesondere galvanisch, verbunden, insbesondere kabelverbunden. Dies ermöglicht eine einfache Verbindung, insbesondere im Unterschied zu einer nicht-erfindungsgemäßen berührungslosen Verbindung. Zusätzlich oder alternativ ist die berührungsbehaftete Verbindung durch die Anordnung des Teilsensors an und/oder in dem Baustofftransportteil nachfolgend der Baustofftrommel ermöglicht. Insbesondere kann der Nebenverbraucher ein Licht, insbesondere einen Arbeitsscheinwerfer, aufweisen, insbesondere sein. Zusätzlich oder alternativ kann die Kontrolleinrichtung zur Kontrolle der Antriebseinrichtung, soweit vorhanden, ausgebildet bzw. konfiguriert sein. Weiter zusätzlich oder alternativ kann die Kontrolleinrichtung einen Computer aufweisen, insbesondere sein. Weiter zusätzlich oder alternativ können der Nebenverbraucher und der Teilsensor zur Versorgung mit Energie durch die Energieversorgungeinrichtung ausgebildet bzw. konfiguriert sein. Weiter zusätzlich oder alternativ kann die Energieversorgungeinrichtung einen elektrischen Energiespeicher, insbesondere eine Batterie, insbesondere einen Akkumulator, und/oder einen Netzanschluss, insbesondere einen Netzstecker, zum Anschluss an ein externes Stromnetz aufweisen, insbesondere sein. Weiter zusätzlich oder alternativ kann die Baustoffvorrichtung, insbesondere das Baustofftransportfahrzeug, die Energieversorgungs- und/oder Datenübertragungseinrichtung aufweisen bzw. umfassen. Im Übrigen wird auf die Fachliteratur verwiesen.

Die erfindungsgemäße Verwendung ist eine, insbesondere automatische, Verwendung mindestens des Teilsensors der Baustoffvorrichtung wie vorhergehend genannt angeordnet an oder in dem Baustofftransportteil der Baustoffvorrichtung zur, insbesondere automatischen, Erfassung mindestens der intensiven Größe oder mindestens der mindestens der intensiven Größe korrespondierenden Größe von dem mindestens teilweise auszuhärtendem Baustoff in dem Baustofftransportteil nachfolgend bzw. nach der Baustofftrommel. Das Baustofftransportteil ist zum Transport des Baustoffs, insbesondere in dem Baustofftransportteil und/oder nachfolgend bzw. nach der Baustofftrommel, ausgebildet. Die Verwendung kann einen Teil oder sogar alle der Vorteile wie vorhergehend für die Baustoffvorrichtung genannt ermöglichen.

### KURZBESCHREIBUNG DER ZEICHNUNGEN

Weitere Vorteile und Aspekte der Erfindung ergeben sich aus den Ansprüchen und aus der Beschreibung von Ausführungsbeispielen der Erfindung, die nachfolgend anhand der Figuren erläutert sind. Dabei zeigen:
- Fig. 1: eine schematische Ansicht einer erfindungsgemäßen Baustoffvorrichtung aufweisend mindestens einen Teilsensor und eine erfindungsgemäße Verwendung mindestens des Teilsensors, insbesondere der Baustoffvorrichtung, und
- Fig. 2: eine weitere schematische Ansicht des Baustofftransportteils der Fig. 1 in Form eines Auslauftrichters.

### DETAILLIERTE BESCHREIBUNG DER AUSFÜHRUNGSBEISPIELE

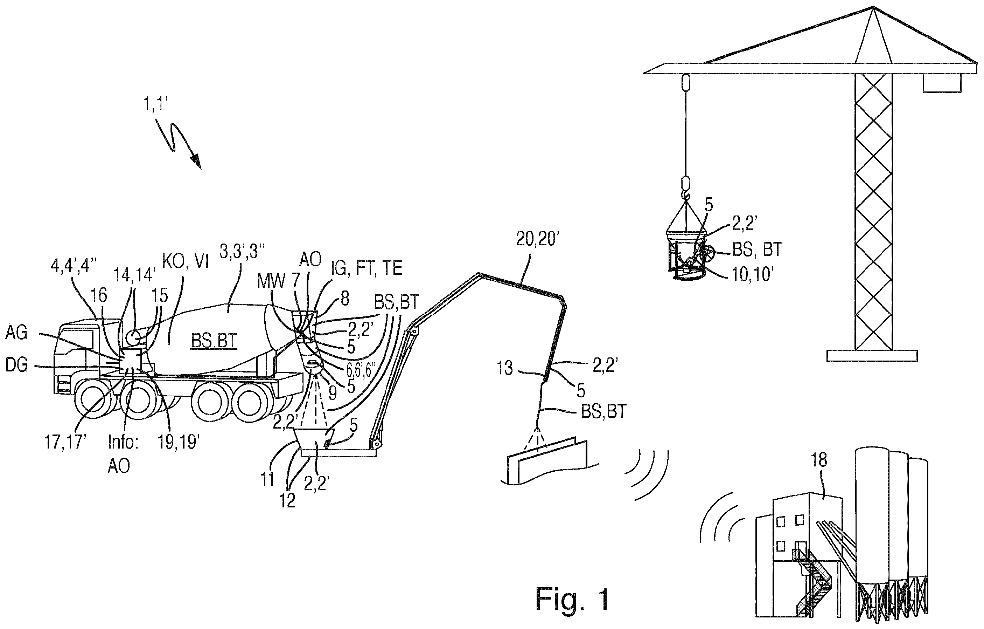

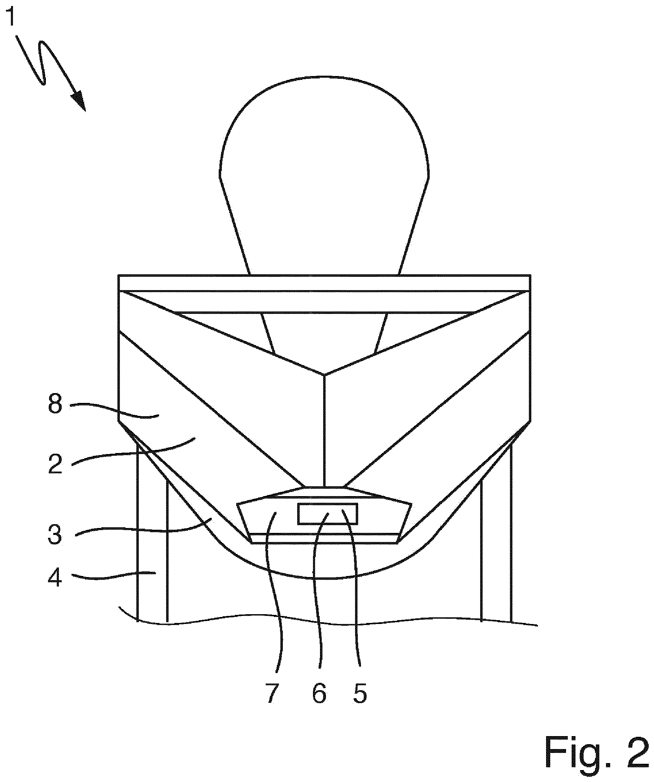

Fig. 1 und 2 zeigen eine erfindungsgemäße Baustoffvorrichtung 1. Die Baustoffvorrichtung 1 weist mindestens ein Baustofftransportteil 2 zur Nachfolge, insbesondere nachfolgend, einer Baustofftrommel 3, insbesondere eines Baustofftransportfahrzeugs 4, und mindestens einen Teilsensor 5 auf. Das Baustofftransportteil 2 ist zum Transport von mindestens teilweise, insbesondere vollständig, auszuhärtendem Baustoff BS ausgebildet, insbesondere transportiert. Mindestens der Teilsensor 5 ist an oder in dem Baustofftransportteil 2 zur Erfassung mindestens einer intensiven Größe IG oder mindestens einer mindestens der intensiven Größe korrespondierenden Größe des Baustoffs BS in dem Baustofftransportteil 2 angeordnet und ausgebildet, insbesondere erfasst.

Fig. 1 zeigt eine erfindungsgemäße Verwendung mindestens des Teilsensors 5 der Baustoffvorrichtung 1 angeordnet an oder in dem Baustofftransportteil 2 der Baustoffvorrichtung 1 zur Erfassung, insbesondere erfassend, mindestens der intensiven Größe IG oder mindestens der mindestens der intensiven Größe korrespondierenden Größe von dem Baustoff BS in dem Baustofftransportteil 2 nachfolgend der Baustofftrommel 3. Das Baustofftransportteil 2 ist zum Transport des Baustoffs BS ausgebildet, insbesondere transportiert.

Im Detail ist die Baustoffvorrichtung 1 eine Betonvorrichtung 1`.

Zusätzlich ist das Baustofftransportteil 2 ein Betontransportteil 2` zur Nachfolge, insbesondere nachfolgend, einer Betontrommel 3` eines Betontransportfahrzeugs 4`.

Weiter zusätzlich ist das Baustofftransportteil 2 zum Transport von auszuhärtendem Beton BT ausgebildet, insbesondere transportiert.

Weiter zusätzlich ist mindestens der Teilsensor 5 zur Erfassung der intensiven Größe IG oder der korrespondierenden Größe des Betons BT ausgebildet, insbesondere erfasst.

Des Weiteren ist mindestens der Teilsensor 5 zur Erfassung einer Feuchtigkeit FT und/oder einer Temperatur TE des Baustoffs BS ausgebildet, insbesondere erfasst.

Außerdem ist mindestens der Teilsensor 5 zur Erfassung der intensiven Größe IG oder der korrespondierenden Größe mittels Mikrowellen MW ausgebildet, insbesondere erfasst.

Weiter weist die Baustoffvorrichtung 1 eine Flachsonde 6, insbesondere eine Tellersonde 6' oder eine Rechtecksonde 6", auf. Die Flachsonde 6 weist den Teilsensor 5 auf.

Zudem weist das Baustofftransportteil 2 ein Prallblech 7 auf. Der Teilsensor 5 ist auf und/oder in dem Prallblech 7 angeordnet.

In dem gezeigten Ausführungsbeispiel ist die Flachsonde 6 auf und/oder in dem Prallblech 7 angeordnet.

Des Weiteren ist in dem gezeigten Ausführungsbeispiel das Baustofftransportteil 2 ein Auslauftrichter 8 des Baustofftransportfahrzeugs 4, insbesondere zur Nachfolge, insbesondere nachfolgend, der Baustofftrommel 3, eine Schurre 9 des Baustofftransportfahrzeugs 4, insbesondere zur Nachfolge, insbesondere nachfolgend, dem Auslauftrichter 8, ein Baustoffkübel 10 in Form eines Krankübels 10', insbesondere zur Nachfolge, insbesondere nachfolgend, dem Auslauftrichter 8 und/oder der Schurre 9, ein Einlauftrichter 11 einer Baustoffpumpe 12, insbesondere zur Nachfolge, insbesondere nachfolgend, dem Auslauftrichter 8 und/oder der Schurre 9, oder ein Endschlauch 13 der Baustoffpumpe 12 oder einer Baustoffverteilervorrichtung 20 in Form eines Baustoffverteilermasts 20`, insbesondere zur Nachfolge, insbesondere nachfolgend, der Baustoffpumpe 12.

Somit weist in dem gezeigten Ausführungsbeispiel die Baustoffvorrichtung 1 fünf Baustofftransporteile 2 auf. In alternativen Ausführungsbeispielen kann die Baustoffvorrichtung, insbesondere entweder, nur ein einziges Baustofftransportteil, vorteilhafterweise den Auslauftrichter, oder zwei, drei, vier oder mindestens sechs Baustofftransporteile aufweisen.

Außerdem weist in dem gezeigten Ausführungsbeispiel die Baustoffvorrichtung 1 fünf Teilsensoren 5 auf. In anderen Worten: Eine Anzahl der Teilsensoren entspricht, insbesondere gleicht, einer Anzahl der Baustofftransportteile. In alternativen Ausführungsbeispielen kann, insbesondere somit, die Baustoffvorrichtung, insbesondere entweder, nur einen Teilsensor oder zwei, drei, vier oder mindestens sechs Teilsensoren aufweisen.

Weiter weist in dem gezeigten Ausführungsbeispiel die Baustoffvorrichtung 1 das Baustofftransportfahrzeug 4 in Form eines Fahrmischers 4", die Baustoffpumpe 12 oder die Baustoffverteilervorrichtung 20 in Form des Baustoffverteilermasts 20' auf. Insbesondere ist die Baustoffvorrichtung 1 das Baustofftransportfahrzeug 4, insbesondere der Fahrmischer 4", und/oder die Baustoffpumpe 12 und/oder die Baustoffverteilervorrichtung 20, insbesondere der Baustoffverteilermast 20'.

In alternativen Ausführungsbeispielen kann die Baustoffvorrichtung, insbesondere entweder, das Baustofftransportfahrzeug, die Baustoffpumpe oder die Baustoffverteilervorrichtung aufweisen, insbesondere sein. Vorteilhafterweise das Baustofftransportfahrzeug.

Zusätzlich weist die Baustoffvorrichtung 1 die Baustofftrommel 3 in Form einer Mischtrommel 3" auf.

Zudem weist die Baustoffvorrichtung 1, insbesondere das Baustofftransportfahrzeug 4, eine Ermittlungseinrichtung 14, insbesondere eine Erfassungseinrichtung 14`, auf. Die Ermittlungseinrichtung 14 ist zur Ermittlung, insbesondere die Erfassungseinrichtung 14' zur Erfassung, einer Konsistenz KO und/oder einer Viskosität VI oder einer der Konsistenz und/oder der Viskosität korrespondierenden Größe des Baustoffs BS, insbesondere in der Baustofftrommel 3, insbesondere einer Antriebsgröße AG einer Antriebseinrichtung 15 zum Drehantrieb der Baustofftrommel 3, ausgebildet, insbesondere ermittelt, insbesondere erfasst.

Insbesondere weist die Baustoffvorrichtung 1, insbesondere das Baustofftransportfahrzeug 4, die Antriebseinrichtung 15 auf.

Des Weiteren weist die Baustoffvorrichtung 1, insbesondere das Baustofftransportfahrzeug 4, eine Bestimmungseinrichtung 16 auf. Die Bestimmungseinrichtung 16 ist zur Bestimmung einer Dosiergröße DG basierend auf mindestens der erfassten intensiven Größe IG oder der erfassten korrespondierenden Größe, insbesondere und der ermittelten Konsistenz KO und/oder der ermittelten Viskosität VI oder der ermittelten korrespondierenden Größe, und einer Information Info über einen Anordnungsort AO des Teilsensors 5 oder eine Art des Baustofftransportteils 2 ausgebildet, insbesondere bestimmt.

Außerdem weist die Baustoffvorrichtung 1, insbesondere das Baustofftransportfahrzeug 4, eine Datenübertragungseinrichtung 17 auf. Die Datenübertragungseinrichtung 17 ist zur, insbesondere kabellosen, Datenübertragung, insbesondere Funk-Datenübertragung, mindestens der erfassten intensiven Größe IG oder der erfassten korrespondierenden Größe, insbesondere und der ermittelten Konsistenz KO und/oder der ermittelten Viskosität VI oder der ermittelten korrespondierenden Größe und/oder der Information Info über den Anordnungsort AO des Teilsensors 5 oder die Art des Baustofftransportteils 2, oder der auf der intensiven Größe IG, insbesondere und der Konsistenz KO und/oder der Viskosität VI, oder der korrespondierenden Größe, insbesondere und der Information Info über den Anordnungsort AO des Teilsensors 5 oder die Art des Baustofftransportteils 2, basierenden Dosiergröße DG, insbesondere zu einer Baustoffmischanlage 18, ausgebildet, insbesondere überträgt.

Weiter weist die Baustoffvorrichtung 1, insbesondere das Baustofftransportfahrzeug 4, einen Nebenverbraucher 19, insbesondere eine Kontrolleinrichtung 19' zur Kontrolle der Baustofftrommel 3, auf. Der Nebenverbraucher 19 und der Teilsensor 5, insbesondere angeordnet an und/oder in dem Baustofftransporteil 2 des Baustofftransportfahrzeugs 4, sind zur Energie- und/oder Datenübertragung, insbesondere durch eine, insbesondere die gemeinsame, Energieversorgungs- und/oder Datenübertragungseinrichtung 17' berührungsbehaftet verbunden, insbesondere kabelverbunden.

Insbesondere weist die Baustoffvorrichtung 1, insbesondere das Baustofftransportfahrzeug 4, die Energieversorgungs- und/oder Datenübertragungseinrichtung 17' auf.

Zusammenfassend und/oder in anderen Worten:
Die Baustoffmischanlage 18 dosiert, mischt und füllt bzw. fördert den Baustoff BS in die Baustofftrommel 3 des Baustofftransportfahrzeugs 4 ein, insbesondere mittels eines Einfüll- bzw. lauftrichters des Baustofftransportfahrzeugs 4 und/oder durch eine Öffnung der Baustofftrommel 3.

Das Baustofftransportfahrzeug 4 transportiert den Baustoff BS in der Baustofftrommel 3 von der Baustoffmischanlage 18 zu einer Baustelle.

Insbesondere auf der Baustelle, füllt bzw. entleert die Baustofftrommel 3 den Baustoff BS in den Auslauftrichter 8. Dabei wird die intensive Größe IG oder die korrespondierende Größe erfasst.

Insbesondere sind an den inneren Wandungen der Baustofftrommel 3 spiralförmige Schaufeln angeordnet. Im Fahrbetrieb bzw. beim Transport ist eine Drehrichtung der Baustofftrommel 3 so, dass der Baustoff nach vorne befördert wird und über die Schaufeln fällt. Insbesondere somit werden ein vorzeitiges Aushärten bzw. Binden des Baustoffs sowie ein Entmischen verhindert. Auf der Baustelle angekommen, wird zum Entleeren die Drehrichtung umgekehrt, so dass der Baustoff nach dem Prinzip der Förderschnecke zu der hoch liegenden Öffnung hinausgefördert wird.

Der Auslauftrichter 8 transportiert den Baustoff BS in die Schurre 9 und/oder den Baustoffkübel 10 und/oder den Einlauftrichter 11 der Baustoffpumpe 12. Dabei wird, insbesondere jeweils, die intensive Größe IG oder die korrespondierende Größe erfasst.

Insbesondere lenkt das Prallblech 7 den Baustoff BS beim Verlassen der Baustofftrommel 3 so, dass der Baustoffkübel 10 optimal befüllt werden kann.

Zusätzlich oder alternativ transportiert die Schurre 9 den Baustoff BS in den Baustoffkübel 10 und/oder den Einlauftrichter 11 der Baustoffpumpe 12. Dabei wird, insbesondere jeweils, die intensive Größe IG oder die korrespondierende Größe erfasst.

Weiter zusätzlich oder alternativ transportiert der Baustoffkübel 10 den Baustoff BS in eine Schalung, insbesondere in welcher der Baustoff BS mindestens teilweise, insbesondere vollständig, aushärtet.

Weiter zusätzlich oder alternativ transportiert die Baustoffpumpe 12 den Baustoff BS in ihren Endschlauch 13 und/oder der Baustoffverteilervorrichtung 20. Dabei wird die intensive Größe IG oder die korrespondierende Größe erfasst.

Weiter zusätzlich oder alternativ transportiert der Endschlauch 13 den Baustoff BS in eine, insbesondere die, Schalung, insbesondere in welcher der Baustoff BS mindestens teilweise, insbesondere vollständig, aushärtet.

Die Erfassung und/oder die Datenübertragung, insbesondere an die Baustoffmischanlage 18, ermöglichen/ermöglicht eine Aussage zur Baustoffqualität des transportierten bzw. ausgebrachten Baustoffs BS und/oder, dass ein Mischmeister und/oder ein Laborant nun auf eventuelle Veränderungen im Baustoff BS reagieren können/kann.

Zusätzlich oder alternativ kann eine Reinigung des Teilsensors 5, insbesondere der Flachsonde 6, nun nach jedem Transport, insbesondere jeder Entleerung, ausgeführt werden bzw. stattfinden.

Wie die gezeigten und oben erläuterten Ausführungsbeispiele deutlich machen, stellt die Erfindung eine vorteilhafte Baustoffvorrichtung aufweisend mindestens einen Teilsensor und eine vorteilhafte Verwendung mindestens eines solchen Teilsensors, insbesondere einer solchen Baustoffvorrichtung, bereit, die jeweils verbesserte Eigenschaften aufweisen.

## Patentansprüche

1. Baustoffvorrichtung (1), wobei die Baustoffvorrichtung (1) aufweist:
- mindestens ein Baustofftransportteil (2) zur Nachfolge einer Baustofftrommel (3), wobei das Baustofftransportteil (2) zum Transport von mindestens teilweise auszuhärtendem Baustoff (BS) ausgebildet ist, und
- mindestens einen Teilsensor (5), wobei mindestens der Teilsensor (5) an oder in dem Baustofftransportteil (2) zur Erfassung mindestens einer intensiven Größe (IG) oder mindestens einer mindestens der intensiven Größe korrespondierenden Größe des Baustoffs (BS) in dem Baustofftransportteil (2) angeordnet und ausgebildet ist,
- wobei die Baustoffvorrichtung (1) eine Betonvorrichtung (1') ist,
- wobei das Baustofftransportteil (2) ein Betontransportteil (2`) zur Nachfolge einer Betontrommel (3') eines Betontransportfahrzeugs (4') ist,
- wobei das Baustofftransportteil (2) zum Transport von auszuhärtendem Beton (BT) ausgebildet ist, und
- wobei mindestens der Teilsensor (5) zur Erfassung der intensiven Größe (IG) oder der korrespondierenden Größe des Betons (BT) ausgebildet ist,
- wobei das Baustofftransportteil (2) ein Auslauftrichter (8) des Baustofftransportfahrzeugs (4), eine Schurre (9) des Baustofftransportfahrzeugs (4), ein Baustoffkübel (10) in Form eines Krankübels (10'), ein Einlauftrichter (11) einer Baustoffpumpe (12) oder ein Endschlauch (13) der Baustoffpumpe (12) oder einer Baustoffverteilervorrichtung (20) in Form eines Baustoffverteilermasts (20') ist,
- **dadurch gekennzeichnet, dass** die Baustoffvorrichtung (1) eine Bestimmungseinrichtung (16) aufweist, wobei die Bestimmungseinrichtung (16) zur Bestimmung einer Dosiergröße (DG) basierend auf mindestens der erfassten intensiven Größe (IG) oder der erfassten korrespondierenden Größe und einer Information (Info) über einen Anordnungsort (AO) des Teilsensors (5) oder eine Art des Baustofftransportteils (2) und einer Anordnungsort- oder Baustofftransportteil-spezifischen Kennformel oder eines Anordnungsort- und/oder Baustofftransportteil-spezifischen Kennfelds ausgebildet ist,
- wobei mindestens der Teilsensor (5) zur Erfassung einer Feuchtigkeit (FT) oder einer Temperatur (TE) des Baustoffs (BS) ausgebildet ist,
- wobei die Baustoffvorrichtung (1) das Baustofftransportfahrzeug (4) in Form eines Fahrmischers (4"), die Baustoffpumpe (12) oder die Baustoffverteilervorrichtung (20) in Form des Baustoffverteilermasts (20') aufweist.

2. Baustoffvorrichtung (1) nach Anspruch 1,
- wobei mindestens der Teilsensor (5) zur Erfassung der intensiven Größe (IG) oder der korrespondierenden Größe mittels Mikrowellen (MW) ausgebildet ist.

3. Baustoffvorrichtung (1) nach einem der vorhergehenden Ansprüche,
- wobei die Baustoffvorrichtung (1) eine Flachsonde (6), insbesondere eine Tellersonde (6') oder eine Rechtecksonde (6"), aufweist, wobei die Flachsonde (6) den Teilsensor (5) aufweist.

4. Baustoffvorrichtung (1) nach einem der vorhergehenden Ansprüche,
- wobei das Baustofftransportteil (2) ein Prallblech (7) aufweist, wobei der Teilsensor (5) auf oder in dem Prallblech (7) angeordnet ist.

5. Baustoffvorrichtung (1) nach einem der vorhergehenden Ansprüche,
- wobei die Baustoffvorrichtung (1) die Baustofftrommel (3) in Form einer Mischtrommel (3") aufweist.

6. Baustoffvorrichtung (1) nach einem der vorhergehenden Ansprüche,
- wobei die Baustoffvorrichtung (1) eine Ermittlungseinrichtung (14) in Form einer Erfassungseinrichtung (14') aufweist, wobei die Ermittlungseinrichtung (14) zur Ermittlung in Form der Erfassungseinrichtung (14') zur Erfassung einer Konsistenz (KO) oder einer Viskosität (VI) oder einer der Konsistenz oder der Viskosität korrespondierenden Größe des Baustoffs (BS) in der Baustofftrommel (3) in Form einer Antriebsgröße (AG) einer Antriebseinrichtung (15) zum Drehantrieb der Baustofftrommel (3) ausgebildet ist.

7. Baustoffvorrichtung (1) nach Anspruch 6,
- wobei die Bestimmungseinrichtung (16) zur Bestimmung der Dosiergröße (DG) basierend auf der ermittelten Konsistenz (KO) oder der ermittelten Viskosität (VI) oder der ermittelten korrespondierenden Größe ausgebildet ist.

8. Baustoffvorrichtung (1) nach einem der vorhergehenden Ansprüche, insbesondere nach Anspruch 6 oder 7,
- wobei die Baustoffvorrichtung (1) eine Datenübertragungseinrichtung (17) aufweist, wobei die Datenübertragungseinrichtung (17) zur, insbesondere kabellosen, Datenübertragung mindestens der erfassten intensiven Größe (IG) oder der erfassten korrespondierenden Größe, insbesondere und der ermittelten Konsistenz (KO) oder der ermittelten Viskosität (VI) oder der ermittelten korrespondierenden Größe oder der Information (Info) über den Anordnungsort (AO) des Teilsensors (5) oder die Art des Baustofftransportteils (2), oder einer auf der intensiven Größe (IG), insbesondere und der Konsistenz (KO) oder der Viskosität (VI), oder der korrespondierenden Größe, insbesondere und der Information (Info) über den Anordnungsort (AO) des Teilsensors (5) oder die Art des Baustofftransportteils (2), basierenden Dosiergröße (DG) für eine Baustoffmischanlage (18) ausgebildet ist.

9. Baustoffvorrichtung (1) nach einem der vorhergehenden Ansprüche,
- wobei die Baustoffvorrichtung (1) mindestens einen Nebenverbraucher (19) in Form einer Kontrolleinrichtung (19') zur Kontrolle der Baustofftrommel (3) aufweist, und
- wobei der Nebenverbraucher (19) und der Teilsensor (5) zur Energie- oder Datenübertragung durch eine gemeinsame Energieversorgungs- oder Datenübertragungseinrichtung (17') berührungsbehaftet verbunden sind.

10. Verwendung mindestens eines Teilsensors (5) einer Baustoffvorrichtung (1) nach einem der vorhergehenden Ansprüche angeordnet an oder in einem Baustofftransportteil (2) der Baustoffvorrichtung (1) zur Erfassung mindestens einer intensiven Größe (IG) oder mindestens einer mindestens der intensiven Größe korrespondierenden Größe von mindestens teilweise auszuhärtendem Baustoff (BS) in dem Baustofftransportteil (2) nachfolgend einer Baustofftrommel (3), wobei das Baustofftransportteil (2) zum Transport des Baustoffs (BS) ausgebildet ist.

## Claims

1. A building material apparatus (1), wherein the building material apparatus (1) comprises:
- at least one building material transport part (2) for following a building material drum (3), wherein the building material transport part (2) is designed to transport building material (BS) to be at least partially cured, and
- at least one part sensor (5), wherein at least the part sensor (5) is arranged on or in the building material transport part (2) and designed to detect at least one intensive variable (IG) or at least one variable, which corresponds to at least the intensive variable, of the building material (BS) in the building material transport part (2),
- wherein the building material apparatus (1) is a concrete apparatus (1'),
- wherein the building material transport part (2) is a concrete transport part (2') for following a concrete drum (3') of a concrete transport vehicle (4'),
- wherein the building material transport part (2) is designed to transport concrete (BT) to be cured, and
- wherein at least the part sensor (5) is designed to detect the intensive variable (IG) or the corresponding variable of the concrete (BT),
- wherein the building material transport part (2) is an outlet hopper (8) of the building material transport vehicle (4), a chute (9) of the building material transport vehicle (4), a building material bucket (10) in form of a crane bucket (10'), an inlet hopper (11) of a building material pump (12) or an end hose (13) of the building material pump (12) and/or of a building material distribution apparatus (20) in form of a building material distribution boom (20'),
- **characterized in that** the building material apparatus (1) comprises a determining device (16), wherein the determining device (16) is designed to determine a metering variable (DG) based on at least the detected intensive variable (IG) or the detected corresponding variable and information (Info) on an arrangement location (AO) of the part sensor (5) or on a type of the building material transport part (2) and an arrangement location- or building material transport part-specific characteristic formula or an arrangement location- and/or building material transport part-specific characteristic map,
- wherein at least the part sensor (5) is designed to detect a moisture (FT) or a temperature (TE) of the building material (BS),
- wherein the building material apparatus (1) comprises the building material transport vehicle (4) in form of a truck mixer (4"), the building material pump (12) or the building material distribution apparatus (20) in form of a building material distribution boom (20').

2. The building material apparatus (1) as claimed in claim 1,
- wherein at least the part sensor (5) is designed to detect the intensive variable (IG) or the corresponding variable by means of microwaves (MW) .

3. The building material apparatus (1) as claimed in one of the preceding claims,
- wherein the building material apparatus (1) comprises a flat probe (6), in particular a disk probe (6') or a rectangular probe (6''), wherein the flat probe (6) comprises the part sensor (5).

4. The building material apparatus (1) as claimed in one of the preceding claims,
- wherein the building material transport part (2) comprises a baffle plate (7), wherein the part sensor (5) is arranged on or in the baffle plate (7).

5. The building material apparatus (1) as claimed in one of the preceding claims,
- wherein the building material apparatus (1) comprises the building material drum (3) in form of a mixing drum (3'').

6. The building material apparatus (1) as claimed in one of the preceding claims,
- wherein the building material apparatus (1) comprises an ascertaining device (14) in form of a detecting device (14'), wherein the ascertaining device (14) is designed to ascertain in form of the detecting device (14') to detect a consistency (KO) or a viscosity (VI) or a variable, which corresponds to the consistency or the viscosity, of the building material (BS) in the building material drum (3) in form of a drive variable (AG) of a drive device (15) for rotationally driving the building material drum (3).

7. The building material apparatus (1) as claimed in claim 6,
- wherein the determining device (16) is designed to determine the metering variable (DG) based on the ascertained consistency (KO) or the ascertained viscosity (VI) or the ascertained corresponding variable.

8. The building material apparatus (1) as claimed in one of the preceding claims, in particular as claimed in claim 6 or 7,
- wherein the building material apparatus (1) comprises a data transmission device (17), wherein the data transmission device (17) is designed for, in particular wireless, data transmission of at least the detected intensive variable (IG) or the detected corresponding variable, in particular and the ascertained consistency (KO) or the ascertained viscosity (VI) or the ascertained corresponding variable or the information (Info) on the arrangement location (AO) of the part sensor (5) or on the type of the building material transport part (2), or a metering variable (DG) based on the intensive variable (IG), in particular and the consistency (KO) or the viscosity (VI), or the corresponding variable, in particular and the information (Info) on the arrangement location (AO) of the part sensor (5) or on the type of the building material transport part (2), for a building material mixing plant (18).

9. The building material apparatus (1) as claimed in one of the preceding claims,
- wherein the building material apparatus (1) comprises at least one auxiliary consumer (19) in form of a control device (19') for controlling the building material drum (3), and
- wherein, for energy or data transmission, the auxiliary consumer (19) and the part sensor (5) are contact-connected by a common energy supply or data transmission device (17').

10. The use of at least one part sensor (5) of a building material apparatus (1) as claimed in one of the preceding claims arranged on or in a building material transport part (2) of the building material apparatus (1) for detecting at least one intensive variable (IG) or at least one variable, which corresponds to at least the intensive variable, of building material (BS) to be at least partially cured in the building material transport part (2) following a building material drum (3), wherein the building material transport part (2) is designed to transport the building material(BS).

## Revendications

1. Dispositif (1) pour matériaux de construction, le dispositif (1) pour matériaux de construction comportant :
- au moins une partie de transport (2) de matériaux de construction qui suit un tambour (3) de matériaux de construction, la partie de transport (2) de matériaux de construction étant réalisée pour le transport de matériaux de construction (BS) devant être au moins en partie durcis, et
- au moins un capteur (5) de partie, au moins le capteur (5) de partie étant disposé et réalisé sur ou dans la partie de transport (2) de matériaux de construction pour détecter au moins une variable intensive (IG) ou au moins une variable des matériaux de construction (BS) correspondant au moins à la variable intensive dans la partie de transport (2) de matériaux de construction,
- le dispositif (1) pour matériaux de construction étant un dispositif (1') pour béton,
- la partie de transport (2) de matériaux de construction étant une partie de transport (2') de béton qui suit un tambour (3') de béton d'un véhicule de transport de béton (4'),
- la partie de transport (2) de matériaux de construction étant réalisée pour le transport de béton (BT) devant être durci, et
- au moins le capteur (5) de partie étant réalisé pour détecter la variable intensive (IG) ou la variable correspondante du béton (BT),
- la partie de transport (2) de matériaux de construction étant une trémie de sortie (8) du véhicule de transport (4) de matériaux de construction, une goulotte (9) du véhicule de transport (4) de matériaux de construction, un godet (10) pour matériaux de construction sous la forme d'un godet de grue (10'), une trémie d'entrée (11) d'une pompe (12) pour matériaux de construction ou un tuyau flexible d'extrémité (13) de la pompe (12) pour matériaux de construction ou d'un dispositif de distribution (20) de matériaux de construction sous la forme d'un mât de distribution (20') de matériaux de construction,
- **caractérisé en ce que** le dispositif (1) pour matériaux de construction comporte un système de définition (16), le système de définition (16) étant réalisé pour définir une variable de dosage (DG) sur la base au moins de la variable intensive (IG) détectée ou de la variable correspondante détectée et d'une information (Info) sur un site d'agencement (AO) du capteur (5) de partie ou un type de la partie de transport (2) de matériaux de construction ou d'une formule caractéristique spécifique à l'emplacement d'agencement ou à la partie de transport de matériaux de construction ou d'un champ caractéristique spécifique à l'emplacement d'agencement et/ou à la partie de transport de matériaux de construction,
- au moins le capteur (5) de partie étant réalisé pour détecter une humidité (FT) ou une température (TE) des matériaux de construction (BS),
- le dispositif (1) pour matériaux de construction comportant le véhicule de transport (4) de matériaux de construction sous la forme d'un camion-malaxeur (4''), la pompe (12) pour matériaux de construction ou le dispositif de distribution (20) de matériaux de construction sous la forme du mât de distribution (20') de matériaux de construction.

2. Dispositif (1) pour matériaux de construction selon la revendication 1,
- au moins le capteur (5) de partie étant réalisé pour détecter la variable intensive (IG) ou la variable correspondante au moyen de micro-ondes (MW).

3. Dispositif (1) pour matériaux de construction selon l'une des revendications précédentes,
- le dispositif (1) pour matériaux de construction comportant une sonde plate (6), en particulier une sonde circulaire (6') ou une sonde rectangulaire (6''), la sonde plate (6) comportant le capteur (5) de partie.

4. Dispositif (1) pour matériaux de construction selon l'une des revendications précédentes,
- la partie de transport (2) de matériaux de construction comportant une chicane (7), le capteur (5) de partie étant disposé sur ou dans la chicane (7).

5. Dispositif (1) pour matériaux de construction selon l'une des revendications précédentes,
- le dispositif (1) pour matériaux de construction comportant le tambour (3) de matériaux de construction sous la forme d'un tambour malaxeur (3'').

6. Dispositif (1) pour matériaux de construction selon l'une des revendications précédentes,
- le dispositif (1) pour matériaux de construction comportant un système de détermination (14) sous la forme d'un système de détection (14'), le système de détermination (14) étant réalisé pour déterminer sous la forme du système de détection (14') pour détecter une consistance (KO) ou une viscosité (VI) ou une variable des matériaux de construction (BS) correspondant à la consistance ou à la viscosité dans le tambour (3) de matériaux de construction sous la forme d'une variable d'entraînement (AG) d'un système d'entraînement (15) pour l'entraînement en rotation du tambour (3) de matériaux de construction.

7. Dispositif (1) pour matériaux de construction selon la revendication 6,
- le système de définition (16) étant réalisé pour définir la variable de dosage (DG) sur la base de la consistance (KO) déterminée ou de la viscosité (VI) déterminée ou de la variable correspondante déterminée.

8. Dispositif (1) pour matériaux de construction selon l'une des revendications précédentes, en particulier selon la revendication 6 ou 7,
- le dispositif (1) pour matériaux de construction comportant un système de transfert de données (17), le système de transfert de données (17) étant réalisé pour le transfert de données, en particulier sans fil, au moins de la variable intensive (IG) détectée ou de la variable correspondante détectée, en particulier de la consistance (KO) déterminée ou de la viscosité (VI) déterminée ou de la variable correspondante déterminée ou de l'information (Info) sur l'emplacement d'agencement (AO) du capteur (5) de partie ou le type de la partie de transport (2) de matériaux de construction, ou d'une variable de dosage (DG) se basant sur la variable intensive (IG), en particulier la consistance (KO) ou la viscosité (VI), ou la variable correspondante, en particulier l'information (Info) sur l'emplacement d'agencement (AO) du capteur (5) de partie ou le type de la partie de transport (2) de matériaux de construction, pour une installation de malaxage (18) de matériaux de construction.

9. Dispositif (1) pour matériaux de construction selon l'une des revendications précédentes,
- le dispositif (1) pour matériaux de construction comportant au moins un consommateur secondaire (19) sous la forme d'un système de commande (19') pour la commande du tambour (3) de matériaux de construction, et
- le consommateur secondaire (19) et le capteur (5) de partie étant reliés avec contact par un système commun d'alimentation en énergie ou de transfert de données (17') pour le transfert d'énergie ou de données.

10. Utilisation au moins d'un capteur (5) de partie d'un dispositif (1) pour matériaux de construction selon l'une des revendications précédentes disposé sur ou dans une partie de transport (2) de matériaux de construction du dispositif (1) pour matériaux de construction pour détecter au moins une variable intensive (IG) ou au moins une variable correspondant au moins à la variable intensive de matériaux de construction (BS) devant être durcis au moins en partie dans la partie de transport (2) de matériaux de construction qui suit un tambour (3) de matériaux de construction, la partie de transport (2) de matériaux de construction étant réalisée pour le transport des matériaux de construction (BS).
